# Europäisches Patentamt

(19) **European Patent Office**

**Office européen des brevets**

(11) Veröffentlichungsnummer: **0 075 727**
**B1**

(12) **EUROPÄISCHE PATENTSCHRIFT**

(45) Veröffentlichungstag der Patentschrift:
07.01.87

(51) Int. Cl.⁴: **C 07 D 213/12**

(21) Anmeldenummer: **82107752.6**

(22) Anmeldetag: **24.08.82**

(54) Verfahren zur Herstellung von 3-Picolin.

(30) Priorität: **29.09.81 CH 6250/81**

(43) Veröffentlichungstag der Anmeldung:
**06.04.83 Patentblatt 83/14**

(45) Bekanntmachung des Hinweises auf die Patenterteilung:
**07.01.87 Patentblatt 87/2**

(84) Benannte Vertragsstaaten:
**AT BE CH DE FR GB IT LI LU NL SE**

(56) Entgegenhaltungen:
**EP - A - 0 040 698**
**EP - A - 0 056 496**
**DE - A - 1 695 296**
**DE - A - 1 965 010**
**DE - A - 2 203 384**
**DE - A - 2 337 087**
**GB - A - 1 240 928**

(73) Patentinhaber: **LONZA AG, Gampel/Wallis (CH)**

(72) Erfinder: **Grayson, James Ian, Dr., Bürchnerstrasse 11, Visp (CH)**
Erfinder: **Dinkel, Rolf, Dr., Lärchenstrasse 8, Münchenstein (CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

## Beschreibung

Die vorliegende Erfindung betrifft ein Verfahren zur Herstellung von 3-Picolin.

Pyridinbasen stellen wichtige Zwischenprodukte in der chemischen Industrie dar, so z. B. bei der Herstellung von Nicotinsäure oder Nicotinsäureamid. Es sind verschiedene Verfahren zur Herstellung von Pyridinbasen bekannt.

2-Methyl-5-äthylpyridin wird heute grosstechnisch im Flüssigphasenverfahren aus Acetaldehyd oder Paraldehyd und Ammoniak in Gegenwart verschiedenster Katalysatoren, wie z. B. Ammoniumsalze, hergestellt. Als Nebenprodukte fallen kleine Mengen an 2- und 4-Picolin an.

2- und 4-Picolin werden heute in Gasphasenreaktionen bei Temperaturen von ca. 400 °C aus Acetaldehyd und Ammoniak unter Verwendung von Festbett- oder Fliessbettkatalysatoren auf Basis Aluminiumsilikat hergestellt.

Für die Erzeugung von Pyridin sowie 3-Picolin, welches immer grössere Bedeutung bekommt, werden heute Gasphasenreaktionen angewendet, wobei durch Zugabe von Formaldehyd zum Acetaldehyd die Bildung von 2- und 4-Picolin zu Gunsten von 3-Picolin unterdrückt wird. Auch diese Umsetzungen finden im Festbett oder Fliessbett mit Aluminiumsilikat als Katalysator bei Temperaturen von etwa 400 °C statt. Nach diesen Verfahren werden Ausbeuten an 3-Picolin in der Grössenordnung von höchstens 40 bis 44% erzielt. Daneben fallen grosse Mengen an Pyridin an.

Es ist auch bekannt, dass anstelle von gesättigten Aldehyden von ungesättigten Aldehyden, wie z. B. Acrolein oder Crotonaldehyd, ausgegangen werden kann. Diese Reaktionen finden in der Gasphase bei hohen Temperaturen statt. Die Ausbeuten liegen im wesentlichen gleich hoch oder aber etwas tiefer als bei der Verwendung von gesättigten Aldehyden als Ausgangsmaterial (DE-OS 2 239 801).

Ebenso ist bekannt, Acrolein zusammen mit einem Ammoniumsalz einer organischen Säure, z. B. Essigsäure, in einem sauren Reaktionsmilieu bei relativ tiefen Temperaturen (15 bis 150 °C) umzusetzen. Die Ausbeuten an 3-Picolin sind relativ niedrig (GP-PS 1 240 928).

Ziel der vorliegenden Erfindung ist es, 3-Picolin in höheren Ausbeuten herzustellen, wobei die Bildung von Pyridin möglichst unterdrückt werden soll.

Erfindungsgemäss wird dies dadurch erreicht, dass man Acrolein oder ein Gemisch von Acrolein und Formaldehyd oder ein Gemisch aus Acrolein, Formaldehyd und Acetaldehyd in flüssiger, wässriger Phase bei Temperaturen von 180 bis 280 °C im geschlossenen Gefäss in Gegenwart von Ammoniak und/oder Ammoniumionen und in Gegenwart von Anionen anorganischer und/oder organischer Säuren, die bei 20 °C eine Säure-Dissoziationskonstante von $10^6$ bis $10^{-12}$ aufweisen, umsetzt.

Unter Acetaldehyd bzw. Formaldehyd im Sinne der Erfindung sind auch dessen Polymere, wie z. B. Paraldehyd, zu verstehen.

Um die für die Reaktion wichtigen Anionen von anorganischen und/oder organischen Säuren, die bei 20 °C eine Säure-Dissoziationskonstante von $10^6$ bis $10^{-12}$ aufweisen, in die Reaktionslösung einzubringen, werden die entsprechenden wasserlöslichen Alkali- und/oder Ammoniumsalze dieser Säuren der Reaktionslösung zugesetzt.

Salze der Säuren im Sinne vorliegender Erfindung sind beispielsweise Natrium-, Kalium- oder Ammoniumsalze
der Pentaborsäure wie Ammoniumpentaborat,
der Kohlensäure wie Ammoniumcarbonat,
der Phosphorsäure wie Kaliumdihydrogenphosphat, Ammoniumdihydrogenphosphat, Dikaliumhydrogenphosphat oder Diammoniumhydrogenphosphat,
der Schwefelsäure wie Natriumhydrogensulfat oder Ammoniumsulfat,
der Fluorsäure wie Natriumfluorid, Ammoniumfluorid oder Ammoniumhydrogendifluorid,
der Salzsäure wie Ammoniumchlorid,
der Bromwasserstoffsäure wie Ammoniumbromid,
der Heptamolybdänsäure wie Ammoniumheptamolybdat,
der Ameisensäure wie Ammoniumformiat,
der Essigsäure wie Natriumacetat oder Ammoniumacetat,
der Propionsäure wie Ammoniumpropionat,
der Buttersäure wie Ammoniumbutyrat,
der Bernsteinsäure wie Dinatriumsuccinat oder Diammoniumsuccinat,
der Adipinsäure wie Diammoniumadipinat,
der Benzoesäure wie Natriumbenzoat oder Ammoniumbenzoat,
der Phthalsäure wie Diammoniumphthalat,
der Terephthalsäure wie Diammoniumterephthalat,
der Nikotinsäure wie Ammoniumnikotinat, sowie
der Isonikotinsäure wie Ammoniumisonikotinat.

Für die Bildung von 3-Picolin aus Acrolein oder einem Gemisch von Acrolein und Formaldehyd oder einem Gemisch von Acrolein, Formaldehyd und Acetaldehyd ist die Anwesenheit von Ammoniak und/oder Ammoniumionen notwendig. Wird Ammoniak angewendet, das entweder gasförmig oder als wässrige Lösung eingesetzt werden kann, genügt es, wenn Alkalisalze der genannten Säuren eingesetzt werden. Es können aber auch Gemische von Alkalisalzen und Ammoniumsalzen eingesetzt werden. Wird auf Ammoniak verzichtet, sind Ammoniumsalze oder Gemische von Ammoniumsalzen und Alkalisalzen zu verwenden. Werden miteinander nicht mischbare flüssige Ausgangsmaterialien, wie z. B. Paraldehyd zusammen mit wässrigem Formaldehyd, verwendet, so ist es vorteilhaft, zur Homogenisierung kleine Mengen Homogenisierungsmittel, wie Alkohole oder cyclischer Äther, einzusetzen, oder die nicht mischbaren flüssigen Ausgangsmaterialien mit je einer eigenen Pumpe in den Reaktor einzuspeisen.

Nach dem Verfahren der Erfindung wird überraschenderweise 3-Picolin in Ausbeuten bis ca. 58,5% erhalten und die Bildung von Pyridin weitgehend unterdrückt (unter 2%). Als Nebenprodukte fallen 3-Äthylpyridin sowie kleine Mengen an 2,5-Dimethylpyridin, 3,5-Dimethylpyridin und 2-Methyl-5-äthylpyridin an.

Gelangen Acrolein und Formaldehyd zusammen zum Einsatz, so wird das Verfahren der Erfindung vorteilhaft mit einem Molverhältnis von 1 zu 0,25 bis 1 zu 0,5 durchgeführt.

Werden Acrolein, Formaldehyd und Acetaldehyd zur Reaktion gebracht, so beträgt das Molverhältnis Acrolein zu Formaldehyd von 1 zu 0,3 bis 1 zu 3 und das Molverhältnis Acrolein zu Acetaldehyd von 1 zu 0,6 bis 1 zu 4.

Die Reaktionstemperaturen liegen vorteilhaft bei 180 bis 280°C, insbesondere bei 205 bis 240°C, vorzugsweise bei 205 bis 230°C.

Die Reaktion wird in flüssiger Phase (wässriger Phase) unter einem Druck, der sich bei der Reaktion im geschlossenen Gefäss bei vorgegebener Temperatur einstellt, durchgeführt. Es ist vorteilhaft, während der Reaktion den Reaktionsansatz zu rühren.

Die Menge Ammoniak und/oder Ammoniumionen liegen vorzugsweise bei 0,5 bis 3 Mol Ammoniak und/oder Ammoniumionen pro Mol Edukt, insbesondere bei 0,5 bis 2,0 Mol pro Mol Edukt.

Die Menge an Anionen von anorganischen und/oder organischen Säuren liegen insbesondere bei 0,1 bis 3 Mol, vorzugsweise bei 0,2 bis 1,0 Mol pro Mol Edukt.

Der Start-pH-Wert der wässrigen Reaktionslösung liegt vorteilhaft zwischen 5,0 und 12,5.

Die Zugabe des Aldehyds erfolgt vorzugsweise nach Massgabe seines Verbrauches. So ist es beispielsweise günstig, beim Arbeiten in einem 2-Liter-Behälter und bei Einsatz von 350 ml Aldehyd diesen kontinuierlich während 20 bis 90 Minuten zuzusetzen. Bei anderen Bedingungen sind die entsprechenden Zugabezeiten zu wählen.

Am Ende der gewünschten Reaktionsperiode wird die Temperatur auf Raumtemperatur gesenkt und das 3-Picolin auf bekannte Weise aus der Reaktionsmischung gewonnen. Eine Methode besteht darin, dass man den pH-Wert der Wasserphase zuerst in den basischen Bereich bringt und dann das organische Material aus der wässrigen Reaktionsmischung mit einem organischen Lösungsmittel, z.B. Benzol, Toluol, Xylol, Methylenchlorid, Chloroform, Äther und dergleichen, extrahiert. Das organische Lösungsmittel wird dann abgedampft und man erhält 3-Picolin durch fraktionierte Destillation. Im Rahmen der Erfindung können auch beliebige andere Methoden zur Abtrennung und Gewinnung des Produktes angewendet werden.

Ein Vorteil des neuen erfindungsgemässen Verfahrens ist auch der, dass die nach der Extraktion der Reaktionsmischung mit einem organischen Lösungsmittel erhaltene wässrige Phase nach Wiederanreicherung mit Ammoniak und/oder Ammoniumionen in den Reaktor zurückgeführt werden kann. Die wässrige Salzphase ist zusammengesetzt aus der ursprünglich in der Salzlösung vorhandenen Wassermenge, der nicht umgesetzten Menge an Ammoniak und/oder Ammoniumsalz, dem gegebenenfalls vorhandenen Metallsalz, der freigesetzten Säure des an der Umsetzung teilnehmenden Ammoniumsalzes sowie einem Mol Wasser für jedes Mol bei der Umsetzung verbrauchten Eduktes. Die wässrige Salzphase wird daher mit Hilfe eines beliebigen bekannten Verfahrens konzentriert, z.B. durch Verdampfen, um das infolge der Kondensationsreaktion gebildete Wasser zu entfernen.

Eine Wiederanreicherung von Ammoniak und/oder Ammoniumsalz wird dann dadurch erreicht, dass man in die wässrige Lösung bei Umgebungstemperatur gasförmigen Ammoniak einbringt, unter Rückbildung des Ammoniumsalzes aus der gegebenenfalls vorhandenen Säure.

Obgleich die Erfindung als diskontinuierliches Verfahren beschrieben worden ist, kann das Verfahren auch im Rahmen der vorliegenden Erfindung kontinuierlich betrieben werden. Bei einer Ausführungsform eines kontinuierlichen Verfahrens werden die Reaktionsteilnehmer kontinuierlich in einen geeigneten Druckreaktor eingeführt, aus dem die Reaktionsmischung kontinuierlich abgezogen wird. Die Reaktionsprodukte werden daraus abgetrennt, die wässrige Salzphase aufkonzentriert und unveränderte Reaktionsteilnehmer werden dann wieder ergänzt und in das Reaktionsgefäss zurückgeführt.

Das kontinuierliche Verfahren kann in jedem Reaktor durchgeführt werden, der eine innige Vermischung der Reaktionsteilnehmer unter heftigem Rühren gestattet, z.B. in einem kontinuierlich gerührten Tankreaktor.

Beispiel 1

1140 ml einer 3,40 molaren wässrigen Lösung von Diammoniumhydrogenphosphat (pH 8,3) wurden in einem 2-Liter-Autoklaven auf 205°C erhitzt und bei 1500 UpM gerührt. In diese Lösung wurde innert 31 Minuten kontinuierlich ein Gemisch aus 115,0 g Acrolein und 100,0 g Äthanol eingepumpt. Dabei variierte der Reaktionsdruck zwischen 20 und 24 bar. Nach beendeter Zugabe des Eduktgemisches wurde die Reaktionsmasse während 20 Minuten bei 205°C weitergerührt und dann auf Raumtemperatur abgekühlt. Schliesslich erfolgte eine Extraktion mit 3mal 100 ml Methylenchlorid sowie eine gaschromatographische Analyse der vereinigten Methylenchloridextrakte, wobei sich folgende Produkte mit den auf eingesetztes Acrolein bezogenen Ausbeuten ergaben:
Pyridin 0,7%, 3-Picolin 50,7%, 3-Äthylpyridin 5,7%, 2,5-Lutidin 0,6%, 3,5-Lutidin 1,0%.

Beispiel 2

1140 ml einer 3,40 molaren wässrigen Lösung von Diammoniumhydrogenphosphat (pH 8,3) wurden in einem 2-Liter-Autoklaven auf 230°C erhitzt und bei 1500 UpM gerührt. In diese Lösung wurden innert 24 Minuten kontinuierlich 115,0 g Acrolein eingepumpt. Dabei variierte der Reaktionsdruck zwischen 32 und 33 bar. Nach beendeter Zugabe des Eduktgemisches wurde die Reaktionsmasse während 10 Minuten bei 230°C weitergerührt und dann auf Raumtemperatur abgekühlt.

Schliesslich erfolgte eine Extraktion mit 3mal 100 ml Methylenchlorid sowie eine gaschromatographische Analyse der vereinigten Methylenchloridextrakte, wobei sich folgende Produkte mit den auf eingesetztes Acrolein bezogenen Ausbeuten ergaben:
Pyridin 0,9%, 3-Picolin 52,4%, 3-Äthylpyridin 7,6%, 2,5-Lutidin 0,1%, 3,5-Lutidin 0,6%, 2-Methyl-5-äthylpyridin 0,3%.

### Beispiele 3–7

Bemerkung zur Berechnung der Ausbeuten: Wenn die Reaktion mit den Ausgangsprodukten Acetaldehyd (oder Paraldehyd) und/oder Formaldehyd und Acrolein durchgeführt wurde, wurde 1 Mol Acrolein als 1 Mol Acetaldehyd + 1 Mol Formaldehyd gerechnet und die Ausbeuten auf total eingesetzten Acetaldehyd (A) oder total eingesetzten Formaldehyd (F) bezogen.

### Beispiel 3

1140 ml einer 3,40 molaren wässrigen Lösung von Diammoniumhydrogenphosphat (pH 8,5) wurden in einem 2-Liter-Autoklaven auf 230 °C erhitzt und bei 1500 UpM gerührt. In diese Lösung wurde innert 54 Minuten kontinuierlich ein Gemisch aus 31,3 g Acrolein, 93,3 g Acetaldehyd und 161,8 g einer 30,1%igen Formaldehydlösung eingepumpt (Molverhältnis Acrolein zu Acetaldehyd zu Formaldehyd = 1 zu 4 zu 3). Dabei variierte der Reaktionsdruck zwischen 31 und 32 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3mal 100 ml Methylenchlorid sowie eine gaschromatographische Analyse der vereinigten Methylenchlorid-Extrakte, wobei sich folgende Produkte mit den je nach Aldehyd-Bedarf bezogenen Ausbeuten ergaben: Pyridin 0,9% (A), 3-Picolin 56,6% (F), 3-Äthylpyridin 24,7% (A), 2,5-Lutidin 3,4% (A), 3,5-Lutidin 0,7% (F), 2-Methyl-5-äthylpyridin 1,6% (A).

### Beispiel 4

Unter den gleichen Bedingungen (Temperatur 230 °C, Dosierzeit 48 Minuten, Reaktionsdruck 32 bis 35 bar) wurden aus 31,3 g Acrolein, 96,0 g Paraldehyd, 160,7 g einer 30,3%igen Formaldehydlösung und 50,0 g Äthanol (kalkulatorisches Molverhältnis Acrolein zu Acetaldehyd zu Formaldehyd = 1 zu 4 zu 3) die folgenden Produkte mit den je nach Aldehyd-Bedarf bezogenen Ausbeuten hergestellt:
Pyridin 1,0% (A), 3-Picolin 54,6% (F), 3-Äthylpyridin 20,3% (A), 2,5-Lutidin 3,8% (A), 3,5-Lutidin 0,7% (F), 2-Methyl-5-äthylpyridin 2,5% (A).

### Beispiel 5

Unter den gleichen Bedingungen (Temperatur 230 °C, Dosierzeit 43 Minuten, Reaktionsdruck 32 bis 36 bar) wurden aus 62,6 g Acrolein, 72,0 g Paraldehyd, 108,2 g einer 30,3%igen Formaldehydlösung und 50,0 g Äthanol (kalkulatorisches Molverhältnis Acrolein zu Acetaldehyd zu Formaldehyd = 1 zu 1,5 zu 1) die folgenden Produkte mit den je nach Aldehyd-Bedarf bezogenen Ausbeuten hergestellt:
Pyridin 1,4% (A), 3-Picolin 56,7% (F), 3-Äthylpyridin 23,7% (A), 2,5-Lutidin 4,2% (A), 3,5-Lutidin 0,8% (F), 2-Methyl-5-äthylpyridin 3,0% (A).

### Beispiel 6

Unter den gleichen Bedingungen (Temperatur 230 °C, Dosierzeit 34 Minuten, Reaktionsdruck 32 bis 35 bar) wurden aus 95,4 g Acrolein, 47,5 g Paraldehyd, 54,7 g einer 30,3%igen Formaldehydlösung und 50,0 g Äthanol (kalkulatorisches Molverhältnis Acrolein zu Acetaldehyd zu Formaldehyd = 3 zu 2 zu 1) die folgenden Produkte mit den je nach Aldehyd-Bedarf bezogenen Ausbeuten hergestellt:
Pyridin 1,5% (A), 3-Picolin 54,5% (F), 3-Äthylpyridin 22,9% (A), 2,5-Lutidin 3,6% (A), 3,5-Lutidin 0,8% (F), 2-Methyl-5-äthylpyridin 2,8% (A).

### Beispiel 7

Unter den gleichen Bedingungen (Temperatur 230 °C, Dosierzeit 42 Minuten, Reaktionsdruck 32 bis 34 bar) wurden aus 125,2 g Acrolein, 52,9 g einer 30,1%igen Formaldehydlösung und 20,0 g Äthanol (Molverhältnis Acrolein zu Formaldehyd = 4 zu 1) die folgenden Produkte mit den je nach Aldehyd-Bedarf bezogenen Ausbeuten hergestellt:
Pyridin 0,5% (A), 3-Picolin 53,3% (A), 3-Äthylpyridin 2,3% (A), 2,5-Lutidin 0,3% (A), 3,5-Lutidin 1,5% (F).

### Beispiel 8

1140 ml einer 3,40 molaren wässrigen Lösung von Ammoniumacetat (pH 7,8) wurden in einem 2-Liter-Autoklaven auf 230 °C erhitzt und bei 1500 UpM gerührt. In diese Lösung wurde innert 57 Minuten kontinuierlich ein Gemisch aus 115,0 g Acrolein und 100,0 g Äthanol eingepumpt. Dabei variierte der Reaktionsdruck zwischen 26 und 30 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3mal 100 ml Methylenchlorid sowie eine gaschromatographische Analyse der vereinigten Methylenchloridextrakte, wobei sich folgende Produkte mit den auf eingesetztes Acrolein bezogenen Ausbeuten ergeben:
Pyridin 0,8%, 3-Picolin 36,3%, 3-Äthylpyridin 6,9%, 2,5-Lutidin 0,6%, 3,5-Lutidin 0,8%, 2-Methyl-5-äthylpyridin 0,3%.

### Beispiel 9

Eine Lösung von 397,1 g Dikaliumhydrogenphosphat in 1140 ml 2,5 molarem wässrigem Ammoniak (pH 12,1) wurde in einem 2-Liter-Autoklaven auf 230 °C erhitzt und bei 1500 UpM gerührt. In diese Lösung wurde innert 67 Minuten kontinuierlich ein Gemisch aus 115,0 g Acrolein und 100,0 g Äthanol eingepumpt. Dabei variierte der Reaktionsdruck zwischen 35 und 38 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3mal 100 ml Methylenchlorid sowie eine gaschromatographische Analyse der vereinigten Methylenchloridextrakte, wobei sich folgende Produkte mit den auf eingesetztes Acrolein bezogenen Ausbeuten ergaben:
Pyridin 1,4%, 3-Picolin 58,4%, 3-Äthylpyridin 4,8%, 2,5-Lutidin 1,2%, 3,5-Lutidin 3,1%, 2-Methyl-5-äthylpyridin 1,0%.

Beispiel 10

Eine Lösung von 137,0 g Natriumhydrogensulfatmonohydrat in 1140 ml 4,0 molarem wässrigem Ammoniak (pH 10,9) wurde in einem 2-Liter-Autoklaven auf 230 °C erhitzt und bei 1500 UpM gerührt. In diese Lösung wurde innert 38 Minuten kontinuierlich ein Gemisch aus 115,0 g Acrolein und 100,0 g Äthanol eingepumpt. Dabei variierte der Reaktionsdruck zwischen 35 und 37 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3mal 100 ml Methylenchlorid sowie eine gaschromatographische Analyse der vereinigten Methylenchloridextrakte, wobei sich folgende Produkte mit den auf eingesetztes Acrolein bezogenen Ausbeuten ergaben:
Pyridin 1,9%, 3-Picolin 56,1%, 3-Äthylpyridin 6,1%, 2,5-Lutidin 1,5%, 3,5-Lutidin 1,1%, 2-Methyl-5-äthylpyridin 0,2%.

## Patentansprüche

1. Verfahren zur Herstellung von 3-Picolin aus Acrolein in Gegenwart von Ammoniak bzw. Ammoniumionen bei erhöhten Temperaturen in der Flüssigphase und unter sauren Bedingungen, dadurch gekennzeichnet, dass man Acrolein oder ein Gemisch von Acrolein und Formaldehyd und gegebenenfalls Acetaldehyd in flüssiger, wässriger Phase bei Temperaturen von 180 bis 280 °C im geschlossenen Gefäss in Gegenwart von Ammoniak und/oder Ammoniumionen und in Gegenwart von Anionen anorganischer und/oder organischer Säuren, die bei 20 °C eine Säure-Dissoziationskonstante von $10^6$ bis $10^{-12}$ aufweisen, umsetzt.

2. Verfahren gemäss Patentanspruch 1, dadurch gekennzeichnet, dass man bei Verwendung eines Gemisches von Acrolein und Formaldehyd ein solches einsetzt, das ein Molverhältnis von 1 zu 0,25 bis 1 zu 0,5 aufweist.

3. Verfahren gemäss Patentanspruch 1, dadurch gekennzeichnet, dass man bei Verwendung eines Gemisches von Acrolein, Formaldehyd und Acetaldehyd ein solches einsetzt, das ein Molverhältnis Acrolein zu Formaldehyd von 1 zu 0,3 bis 1 zu 3 und ein Molverhältnis Acrolein zu Acetaldehyd von 1 zu 0,6 bis 1 zu 4 aufweist.

4. Verfahren gemäss Patentansprüchen 1 und 3, dadurch gekennzeichnet, dass man Acetaldehyd als solches oder in Form seiner Derivate anwendet.

5. Verfahren gemäss Patentansprüchen 1 bis 4, dadurch gekennzeichnet, dass man die Anionen der anorganischen und/oder organischen Säuren durch Zusatz der entsprechenden wasserlöslichen Alkali und/oder Ammoniumsalze in die Reaktionslösung einbringt.

6. Verfahren gemäss Patentansprüchen 1 bis 5, dadurch gekennzeichnet, dass man bei Temperaturen von 205 bis 230 °C arbeitet.

7. Verfahren gemäss Patentansprüchen 1 bis 6, dadurch gekennzeichnet, dass man die Salze in wässriger Lösung in einer Konzentration von 0,3 bis 10 Mol/Liter anwendet.

## Claims

1. Process for preparing 3-picoline from acroleine in the presence of ammonia and ammonium ions resp. at elevated temperatures in the liquid phase and under acidic conditions, characterized in that acroleine or a mixture of acroleine and formaldehyde and, if desired, acetaldehyde is reacted in liquid aqueous phase at temperatures of 180 to 280 °C in a closed vessel in the presence of ammonia and/or ammonium ions and in the presence of anions of inorganic and/or organic acids showing an acid dissociation constant of $10^6$ to $10^{-12}$ at 20 °C.

2. Process according to patent claim 1, characterized in that when using a mixture of acroleine and formaldehyde such mixture shows a molar ratio of 1:0.25 to 1:0.5 is employed.

3. Process according to patent claim 1, characterized in that when using a mixture of acroleine, formaldehyde and acetaldehyde such mixture shows a molar ratio of acroleine to formaldehyde of 1:0.3 to 1:3 and a molar ratio of acroleine to acetaldehyde of 1:0.6 to 1:4 is employed.

4. Process according to patent claims 1 and 3, characterized in that acetaldehyde is used as such or in the form of its derivatives.

5. Process according to patent claims 1 to 4, characterized in that the anions of inorganic and/or organic acids are introduced into the reaction solution by adding the respective water-soluble alkali and/or ammonium salts.

6. Process according to patent claims 1 to 5, characterized in that one operates at temperatures of 205 to 230 °C.

7. Process according to patent claims 1 to 6, characterized in that the salts are used in aqueous solution in a concentration of 0.3 to 10 mols/l.

## Revendications

1. Procédé pour la préparation de la 3-picoline à partir de l'acroléine en présence d'ammoniac ou d'ions ammonium à des températures augmentées en phase liquide et dans des conditions acides, caractérisé en ce que l'on fait réagir de l'acroléine ou un mélange d'acroléine et de formaldéhyde et éventuellement d'acétaldéhyde en phase aqueuse, liquide à des températures de 180 à 280 °C dans un récipient fermé en présence d'ammoniac et/ou d'ions ammonium et en présence d'anions d'acides minéraux et/ou organiques qui présentent à 20 °C une constante de dissociation acide de $10^6$ à $10^{-12}$.

2. Procédé selon la revendication 1, caractérisé en ce que lorsque l'on utilise un mélange d'acroléine et de formaldéhyde, on met en œuvre un mélange tel qu'il présente un rapport molaire de 1 à 0,25 jusqu'à 1 à 0,5.

3. Procédé selon la revendication 1, caractérisé en ce que lorsque l'on utilise un mélange d'acroléine, de formaldéhyde et d'acétaldéhyde, on met en œuvre un mélange qui présente un rapport molaire de l'acroléine au formaldéhyde de 1 à 0,3 jusqu'à 1 à 3 et un rapport molaire de l'acroléine à l'acétaldéhyde de 1 à 0,6 jusqu'à 1 à 4.

4. Procédé selon les revendications 1 et 3, caractérisé en ce que l'on utilise de l'acétaldéhyde tel quel ou sous forme de ses dérivés.

5. Procédé selon les revendications 1 à 4, caractérisé en ce qu'on introduit les anions des acides minéraux et/ou organiques dans la solution réactionnelle par addition des sels alcalins et/ou d'ammonium solubles dans l'eau correspondants.

6. Procédé selon les revendications 1 à 5, caractérisé en ce que l'on travaille à des températures de 205 à 230 °C.

7. Procédé selon les revendications 1 à 6, caractérisé en ce que l'on utilise les sels en solution aqueuse à une concentration de 0,3 à 10 moles/litres.